# EUROPEAN PATENT SPECIFICATION

(11) **EP 3 185 770 B1**
(45) Date of publication and mention of the grant of the patent: **07.04.2021**
(21) Application number: 15756145.7
(22) Date of filing: 25.08.2015
(51) Int. Cl.: A61B 5/11

(54) **COMFORT BAND FOR WEARING WITH AN ELECTRONIC SURVEILLANCE BRACELET**
KOMFORTBAND ZUM TRAGEN MIT EINEM ELEKTRONISCHEN ÜBERWACHUNGSARMBAND
BANDE DE CONFORT À PORTER AVEC UN BRACELET DE SURVEILLANCE ÉLECTRONIQUE

(30) Priority: 26.08.2014 CH 12752014
(43) Date of publication of application: 05.07.2017
(73) Proprietor: Geosatis SA, 2340 Le Noirmont (CH)
(72) Inventor: FERNANDES DEMETRIO, José Carlos, CH-2338 Les Emibois (CH); HUNKELER, Urs, CH-5412 Vogelsang (CH); DURAND, Anita, CH-2300 La Chaux-de-Fonds (CH); PRAPLAN, Vincent, CH-2300 La Chaux-de-Fonds (CH); POTURALSKI, Marcin, CH-1022 Chavannes (CH)
(74) Representative: reuteler & cie SA
(86) International application number: PCT/EP2015/069397
(87) International publication number: WO 2016/030349

(56) References cited:
- EP-A1- 2 608 156
- WO-A1-02/33620
- DE-A1-102012 203 049
- US-A- 5 669 390
- US-A1- 2009 177 131
- US-A1- 2011 201 960

## Description

### Technical Field

The present invention relates to accessories for electronic surveillance bracelets, in particular a comfort band for such a bracelet.

### State of the art

Presently, electronic surveillance bracelets are often used as an alternative to incarceration for persons convicted of a crime, or as an alternative to remanding an individual into custody while awaiting trial. Such bracelets are typically worn around the lower leg or the ankle, and utilise a satellite positioning system such as GPS (Global Positioning System) or GNSS (Global Navigation Satellite System) to determine the location of the wearer. This location is communicated to the relevant authorities.

To prevent unauthorised removal of the bracelet, the bracelet is typically constructed of rigid shells which provide a degree of resistance to cutting, and incorporate various systems for detecting tampering and attempted removal. When tampering is detected, this is indicated to the relevant authorities. An example of such an electronic security bracelet is disclosed in the patent application EP 2 608 156 in the name of the present applicant.

However, such rigid bracelets can be uncomfortable for the wearer, particularly if the wearer participates in sport or exercise requiring vigourous movement of the limb to which the bracelet is attached. In particular, the hard shell can impinge on the ankle or foot in an uncomfortable and possibly even painful manner. A standard solution often proposed to wearers is to wrap the bracelet in the cuff and the leg of a sock worn at the same time, which eliminates bouncing of the bracelet during exercise, yet causes it to apply pressure to the ankle or foot.

Document DE 10 2012 203 049 describes a cuff for a sphygmomanometer with an outer sheath containing the usual inflatable air cushion for taking blood pressure measurements. Also contained within this outer sheath is a weighted portion intended to help the user to be able to correctly position the sphygmomanometer cuff without needing particular attention. Such an arrangement is unpadded and clearly does not provide a solution for improving the comfort of a rigid surveillance bracelet worn around the ankle, particularly in respect of avoiding contact between the lower end face of the bracelet and the foot or ankle.

Document US 5,669,390 describes a single-use protective barrier medical accessory for isolating a sphygmomanometer cuff from a patient, to prevent contamination of the cuff. This barrier is formed as an impermeable sleeve, open at both ends, which is placed on the patent's arm before the cuff is applied. If desired, one end of the sleeve can be folded down to cover the cuff. Again, such an arrangement would not help in any way in respect of the comfort of a wearer of a rigid surveillance bracelet, since it is entirely unpadded. Indeed, long-term wearing of such a sleeve in combination with an electronic surveillance bracelet is likely to be objectionable due to the impermeable nature of the cuff, which would lead to unpleasant sweating of the lower leg and ankle and accompanying risk of skin complaints.

Document US 2011/201960 describes a padded wristband sensor comprising sensors for measuring physiological parameters of a wearer. While such an arrangement could in principle be worn between a rigid electronic surveillance bracelet and the leg of the wearer, it has a substantially constant thickness. Hence, it would only cushion the leg from the interior wall of the bracelet and would do nothing to prevent uncomfortable contact between the ankle or foot and the lower end face of the bracelet, which is the primary concern in the present invention. Furthermore, due to the constant thickness, there is a risk that the band could ride up the leg of the wearer and out of contact with the bracelet.

A first object of the invention is thus to provide an accessory for such an electronic surveillance bracelet which overcomes the above-mentioned disadvantages.

In addition, it can be advantageous to incorporate physiological sensors into such electronic surveillance bracelets for monitoring the health conditions of the wearer. Such sensors can, for instance, measure heart rate, blood oxygenation, transpiration, skin temperature and so on. However, with such a rigid bracelet it is difficult to keep physiological sensors in contact with the skin of the wearer, which is necessary for making accurate physiological measurements. As such, a further object of the invention is to overcome this disadvantage.

### Disclosure of the invention

More precisely, the invention relates to a kit of parts comprising an electronic surveillance bracelet (11) and a comfort band for wearing between the electronic surveillance bracelet and a leg of a wearer. This comfort band comprises a tubular elastic element adapted to retain the comfort band on the leg of the wearer, and a cushion provided along an edge of the elastic element which is intended to be disposed towards the foot of the wearer, the cushion extending outwardly from the elastic element and being arranged so as to form an abutment for an end face of the electronic surveillance bracelet.

In consequence, direct contact between the end face of the electronic surveillance bracelet and the foot or ankle of the wearer can be eliminated by the outwardly-extending cushion being interposed between the bracelet and the foot or ankle, improving comfort for the wearer.

Advantageously, the elastic element has a height at least corresponding to the height of the electronic surveillance bracelet, eliminating all direct contact between the electronic surveillance bracelet and the wearer, further increasing comfort.

Advantageously, the elastic element forms a lip extending under the cushion, the lip being arranged to urge the cushion in an outward direction. This lip thus prevents folding under or bunching up of the cushion under the elastic element.

Advantageously, the comfort band comprises a closure to permit the comfort band to be opened and re-closed, the closure preferably comprising a hook and loop fastener. The comfort band can thus be easily put on and taken off without requiring to be passed over the foot of the wearer, and the circumference of the comfort band can be easily adjusted. As a further consequence, the comfort band can be applied without removing footwear.

Advantageously, at least one of the elastic element and the cushion comprises at least one physiological property sensor arranged to be in contact with the skin of the wearer. Good contact between the physiological property sensor and the skin of the wearer is thus achieved. The physiological property sensor may comprise at least one of:
- a heart rate sensor;
- a blood oximetry sensor;
- a skin temperature sensor;
- a perspiration sensor;
- a chemical sensor for detecting a foreign substance such as (but not limited to) alcohol, pharmaceuticals or drugs;
- an identification sensor, such as (but not limited to) an ultrasonic sensor for measuring a property of the structure of the wearer's ankle.

Advantageously, the comfort band further comprises at least one electrical contact pad adapted to cooperate with a corresponding electrical contact provided on the electronic surveillance bracelet. Communication between the electronic surveillance bracelet and the physiological property sensor in the comfort band is thus assured. Advantageously, the electrical contact pad is provided on a surface of the cushion arranged to face an end surface of the electronic surveillance bracelet, such that the effect of gravity and the swinging of the wearer's leg pulls the electronic surveillance bracelet into contact with the cushion, ensuring reliable electrical connection.

Alternatively or additionally, the electrical contact pad can be provided on an outward-facing surface of the elastic element, which permits a greater surface area for the electrical contact pad.

Advantageously, the cushion is shaped so as to attain an alignment between the electrical contact pad provided on the comfort band and the corresponding electrical contact provided on the electronic surveillance bracelet. Reliable electrical contact is thus promoted.

Alternatively or additionally, the comfort band may comprise at least one first antenna adapted to communicate with a corresponding second antenna provided on or in the electronic surveillance bracelet. The first antenna is preferably adapted to transmit data from the physiological property sensor to the electronic surveillance bracelet, and/or to power the physiological property sensor wirelessly. Wireless communication and/or wireless powering of the physiological property sensor is thus achieved.

Advantageously, the cushion is shaped so as to attain an alignment between the first antenna provided on the comfort band and the second antenna provided on or in the electronic surveillance bracelet. Good wireless reception between the antennas is thus promoted.

Advantageously, the cushion comprises at least one thicker section and at least one thinner section arranged such that an electronic surveillance bracelet achieves said alignment as a result of movement of the wearer's leg.

### Brief description of the drawings

Further features of the invention are explained in more detail in the following description in reference to the following figures, in which:
- Figure 1 shows a schematic perspective view of a comfort band according to the invention;
- Figure 2 shows a schematic perspective view of a variant of a comfort band according to the invention;
- Figure 3 shows a schematic perspective view from underneath of a the comfort band of figure 1;
- Figure 4 shows a cross-sectional through the comfort band of figure 1;
- Figure 5 shows a schematic perspective view of the comfort band in use;
- Figures 6a - 6c show cross-sectional views of embodiments of a "connected" comfort band in combination with an electronic surveillance bracelet according to the invention;
- Figure 7a shows a schematic horizontal cross-section through a phone element of a particular embodiment of a comfort band; and
- Figures 7b and 7c show two variants of schematic partial cutaway views along A-A of figure 7a.

### Embodiments of the invention

In the following description, directional indications are given with respect to the orientation of the comfort band in normal use, worn around or just above the ankle with the wearer standing upright. As such, "lower", "downward" and similar indicate a direction towards the wearer's foot, "upper", "upwards" and similar indicate a direction towards the wearer's knee, "inwards", "inner" and similar indicate a direction towards the inside of the comfort band, and vice versa.

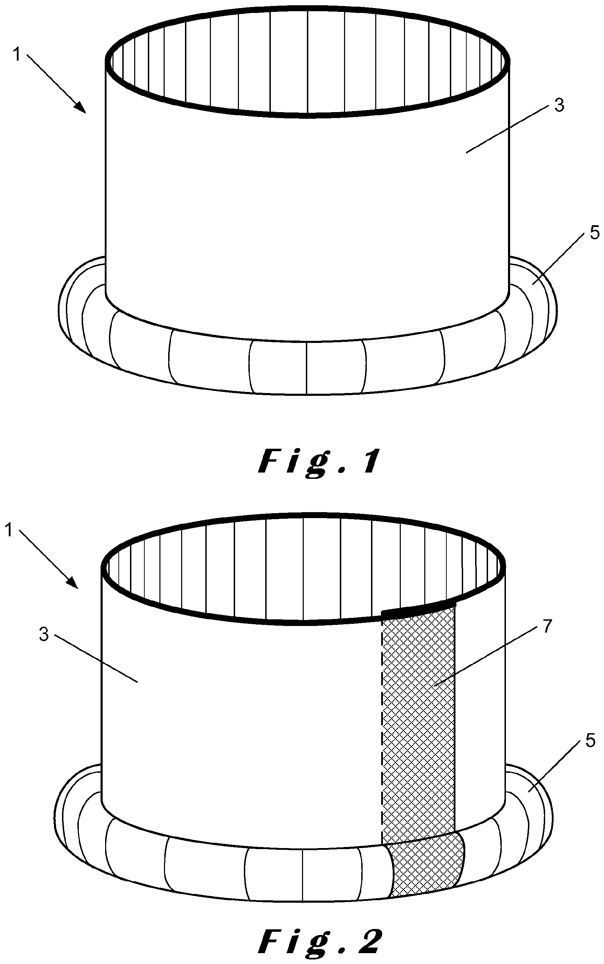

Figure 1 illustrates a schematic perspective view of a comfort band 1 according to the invention. This comfort band 1 comprises an elastic element 3 which is formed into a ring and sized to fit snugly around the leg of a wearer. Typically, this elastic element 3 is made from a strip of elasticated material such as is commonly used in the manufacture of clothing, such as for waistbands, cuffs, and so on. Alternatively, elastic element 3 can be made of neoprene or any other convenient material. Attached at an edge of the elastic element 3 is a cushion 5, which extends around the edge of the elastic element 3 and is thus also formed into a ring.

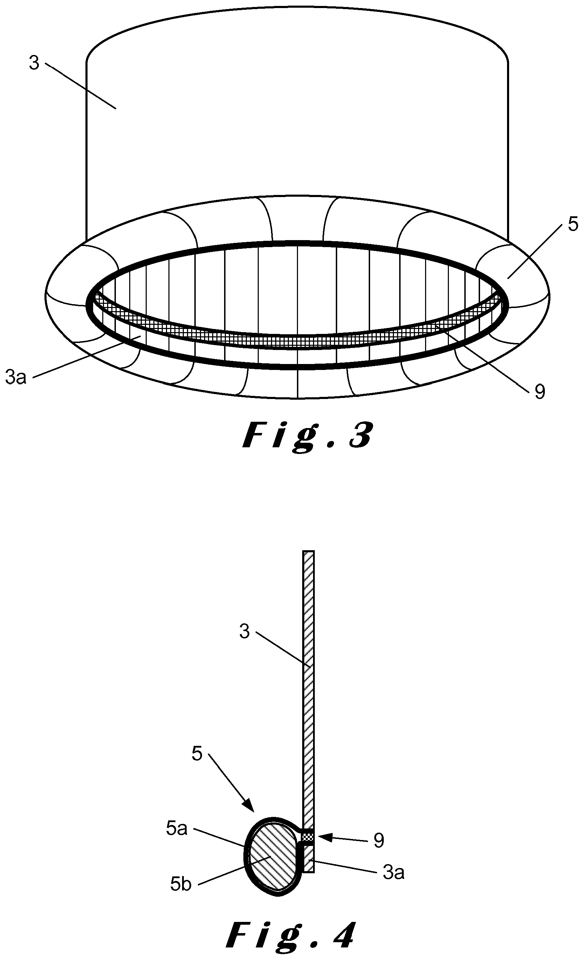

Cushion 5 can take any convenient form. For instance, it can be formed of a shell of material 5a (see figure 4) in which is placed an elongated piece of foam 5b (see figure 4) or other packing material, of any convenient cross-section such as circular, oval, square, rectangular or so on. In the case of an elastic element 3 made of neoprene, cushion 5 can also be integrally formed as one piece with the elastic element 3, or equally may be stitched thereto.

In figure 1, the comfort band 1 is shown as a slip-on band which is not openable. Such a comfort band 1 must be pulled on over the foot of the wearer, and is not adjustable. As such, the elastic element 3 is either woven as a tube, or is formed into a tube by sewing or bonding a strip of material along its edges. Such a slip-on band can be made extremely thin, and thus may provide the highest level of comfort.

Figure 2, on the other hand, shows a modified comfort band 1 in which the band is secured by means of a closure 7 which, in the illustrated form, is a hook-and-loop-type closure, commonly known by the trade name Velcro, which secures the overlapping ends of the comfort band 1. Naturally, any other convenient type of closure can also be used, although a hook-and-loop closure is preferred for comfort since it is very supple and thus comfortable. Furthermore, by providing such a closure, the circumference of the comfort band 1 can be varied, which is not possible with a slip-on-type band, however the closure 7 adds thickness and stiffness to the band compared to the comfort band 1 of figure 1. Additionally, such a band can be placed around the leg of a wearer without removing any footwear present.

Figures 3 and 4 illustrate a particular embodiment of attaching the cushion 5 to the elastic element 3. In this embodiment as illustrated, the cushion 5 is constituted by enclosing a rod of foam material 5b in a shell 5a formed of fabric tape. The edges of the fabric tape are then sewed by means of stitching 9 along the edge of the elastic element 3 such that a lip 3a of the material of the elastic element 3 remains. This lip 3a of material applies sufficient outwards pressure on the cushion 5 that cushion 5 is prevented from folding under the elastic element 3 in use. One way to achieve this is to position the two edges of the fabric tape which form the shell 5a together with the edge of the elastic element 3 all pointing in the same direction with the foam material 5b being positioned towards the opposite edge of the elastic element 3, and to sew all three edges using a relatively wide stitching 9 such as a zigzag stitch, the material defined by the width of the zigzag stitch together with the excess material of the elastic element 3 protruding beyond the stitching 9 thereby forming the lip 3a. Although the excess material should be kept to a minimum, for instance up to 1, 2, 3, 4 or 5 millimetres beyond the stitching 9, it may extend for several centimetres, for instance up to 2 or 3 centimetres. In such a latter case, the cushion 5 is still considered to be situated along the lower edge of the elastic element 3, irrespective of where it is stitched, since in use the cushion 5 will be in any case positioned here.

Naturally, other means of forming this lip are possible, and it is not obligatory that material be stitched: depending on the materials chosen, it may be glued, riveted, welded, or so on. In the case of a single piece construction of neoprene or other similar suitable material, the lip 3a may be formed integrally.

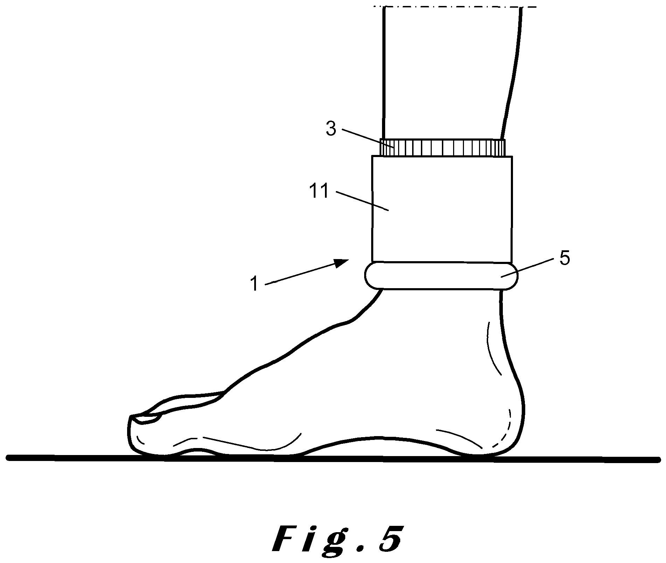

Figure 5 shows schematically comfort band 1 in use. As is generally known, a security bracelet 11 is worn around the ankle or lower leg of the wearer, as illustrated. Comfort band 1 is worn around the ankle or lower leg underneath the security bracelet 11. As is clearly shown, cushion 5 is thus interposed between the lower face of the security bracelet 11 and the ankle/foot of the wearer, and elastic element 3 is interposed between the inside face of security bracelet 11 and the wearer's skin. Although it is not strictly necessary, it is advantageous that elastic element 3 is at least as tall, preferably taller, than the security bracelet 11 such that elastic element 3 protrudes above it as can be seen in figure 5. This minimises contact between the security bracelet 11 and the wearer's skin, and provides the maximum comfort.

As discussed briefly in the introduction, it is often desirable to be able to perform physiological measurements in conjunction with the use of an electronic security bracelet. There are numerous non-invasive physiological measurements that can be carried out at the skin either by measuring electrical signals, electrical resistance, or making optical or ultrasound measurements. Nonlimiting examples of such measurements are pulse oximetry for measuring blood oxygen content, heart rate, skin temperature, perspiration by means of skin conductivity, presence of blood-borne foreign substances such as alcohol, pharmaceuticals, drugs, and so on. Such measurements can be useful for remotely checking aspects of the health and physical condition of the wearer. For instance, an alcohol or drug sensor can determine whether a wearer has been drinking or taking drugs, or a pharmaceutical detector can determine whether a wearer has been taking certain medication that he or she is required to take, such as cardiac medication or mood-altering pharmaceuticals. Another example of a physiological sensor is a biometric sensor using ultrasound, for carrying out intracorporeal imagery. Such a sensor can perform an echography of the bones of the wearer's ankle or lower leg, in particular of the tibia and fibula, and can be used at low resolution to determine that the wearer has not removed the security bracelet and placed it on an animal or other mobile object. At high resolution, the sensor could be used to identify the wearer based on unique features of the bones imaged. Other forms of identification sensor are also possible.

However, in the case of rigid security bracelets, it is difficult to keep the sensors in contact with the skin where they need to be in order to accurately perform measurements, since such a bracelet cannot be sufficiently close-fitting.

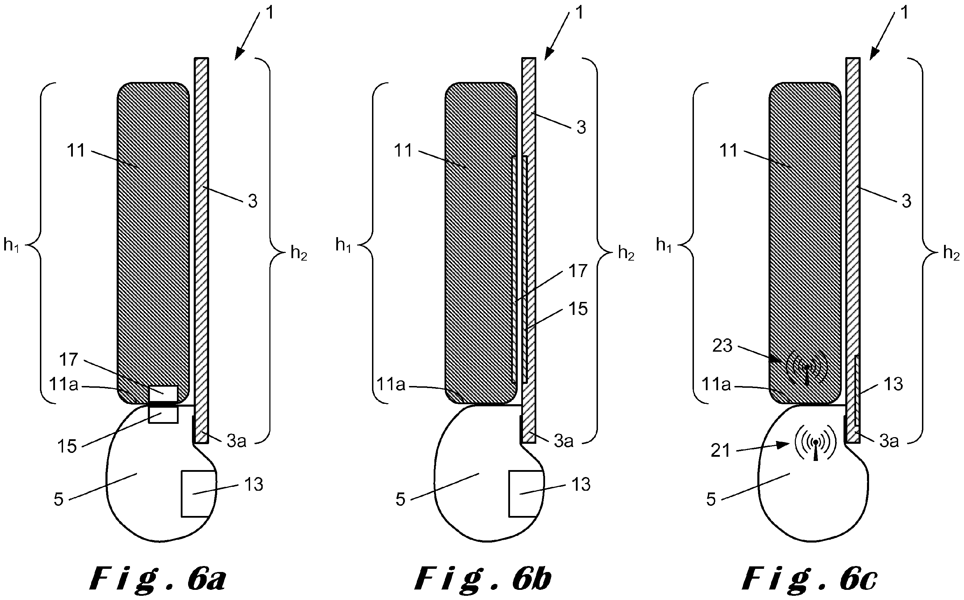

Figures 6a to 6c present solutions to this problem, each of which is illustrated comfort band 1 in combination with an electronic security bracelet 11. In figure 6a, at least one physiological sensor 13 of any type including but not limited to those discussed above is integrated into cushion 5 such that, when the comfort band 1 is worn, physiological sensor 13 is situated on an inward-facing surface of the cushion 5 and is thus brought into contact with the skin of the wearer. Alternatively, physiological sensor 13 may simply be attached on the inward-facing surface of the cushion 5. As many physiological sensors 13 as desired may be incorporated into the cushion 5 as desired. It is also possible to incorporate one or more batteries into cushion 5, however it is desirable to power the sensor from in these variants.

In order to power the physiological sensor 13 and to receive signals produced thereby, the cushion 5 comprises an electrical contact pad 15 in electrical communication with one or more physiological sensors 13. Electrical contact pad 15 is situated on an outward-facing upper surface of the cushion 5 such that it can be brought into contact with a corresponding electrical contact 17 situated on the lower, downwards-facing end of electronic security bracelet 11. The electrical contact pad 15 and electrical contact 17 are thus maintained in contact by gravity, ensuring reliability of connection. It is also possible to incorporate one or more batteries for accumulators into cushion 5, if required. Comfort band 1 is thus a so-called "connected" comfort band.

Electrical contact pad 15 may be constituted by a monolithic metallic or conductive polymer pad, by wires interwoven with the material constituting the exterior of the cushion 5, or any other convenient arrangement. Naturally, as many electrical contact pads 15 as desired may be incorporated. To maximise the electrical contact, either or each of the electrical contact pad 15 and the electrical contact 17 may be elongated and extend around the respective component for a certain angle such as 45°, 90°, 180° or even greater.

Figure 6b shows an arrangement which differs from that of figure 6a in that electrical contact pad 15 is provided on an outward-facing surface of the elastic element 3, and the corresponding electrical contact 17 is provided on an inward-facing surface of the electronic security bracelet 11. In order to maximise the probability of maintaining good contact between electrical contact pad 15 and corresponding electrical contact 17, each may be arranged diagonally on their respective component, at opposite angles.

Figure 6c illustrates a variant in which physiological sensor 13 is integrated into the elastic element 3 rather than the cushion 5, and in which power is supplied to the physiological sensor 13 and/or signals are received therefrom wirelessly, by means of a first antenna 21 is situated in the cushion 5, which communicates with a second antenna 23 situated on or in the electronic security bracelet 11. The required circuitry and any required batteries or accumulators (not illustrated) are integrated into cushion 5. Antenna 21 may alternatively be integrated into elastic element 3. Such wireless power and data transfer technology is well understood (for instance in the field of RFID tags) and need not be described further here. The various antennas 21, 23 may each be provided around the whole of the periphery of the cushion 5 or elastic element 3 and the electronic security bracelet 11, or one or both of them may be a smaller antenna situated over a smaller area.

It should be noted that the features of the variance illustrated in figures 6a-c can be combined in any convenient manner, and that physiological sensors 13, electrical contact pads 15, first antennas 21 and so on can be positioned at will on any convenient part of the comfort band 1, in combination or separately.

As has already been discussed briefly in connection with figure 5, it can more clearly be seen from figures 6a-c that the height h2 of the elastic element 3 considered parallel to its central axis is advantageously greater than the height h1 of the electronic security bracelet 11, such that the upper edge of the elastic element 3 protrudes above the upper face of the electronic security bracelet 11.

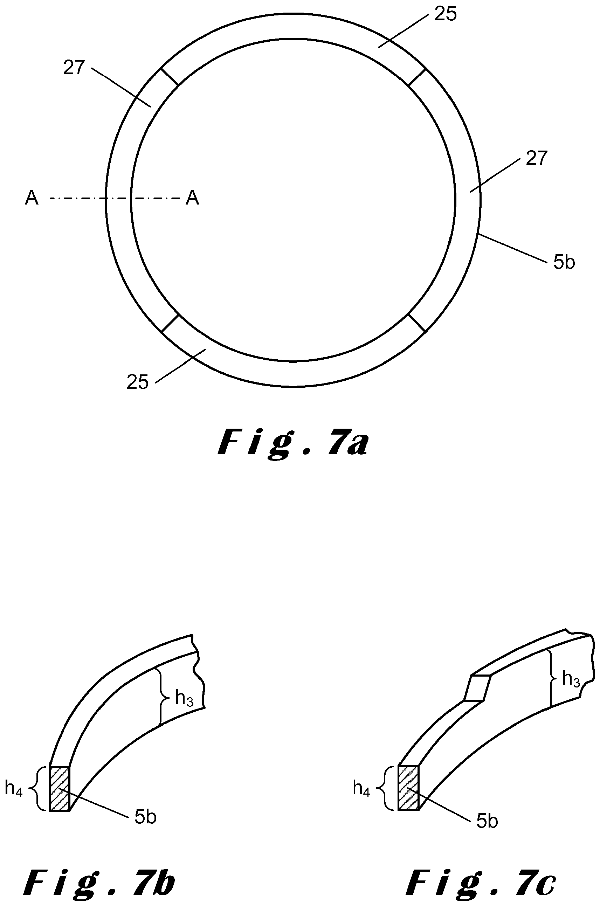

In the case of the "connected" band 1 as illustrated in figures 6a-c, it is advantageous that the alignment between the comfort band 1 and the electronic security bracelet 11 be maintained such that contact is maintained as far as possible between the various electrical contacts 15, 17, and/or that wireless communication is maintained between the antennas 21, 23. As can be seen in figures 7a-c, to this end, the foam 5b of the cushion 5 (or the entirety of the cushion 5 itself in the case that this latter is formed as a single piece) comprises at least one thicker region 25 with a height h3 (considered parallel to the central axis of the elastic element 3) which is greater than the height h4 of diametrically opposed thinner region 27 interposed between the thicker regions 25. In the illustrated variant, a pair of diametrically opposed thicker regions 25 are provided, between which are interposed a corresponding pair of diametrically opposed thinner regions 27.

In figure 7b, the height of the foam 5b varies continuously between h3 and h4, whereas in figure 7c, the foam 5b is of continuous height h3, h4c in each of the thicker and thinner regions 25, 27 respectively, with a relatively abrupt step change between these regions.

This varying height has the result that if the security bracelet 11 is oval, and/or has a height which varies around its circumference in a complimentary fashion to that of the foam 5b, and/or the lower end face 11a of the security bracelet 11 comprises at least one appropriately-arranged protuberance, normal movement of the leg of the wearer will cause the security bracelet 11 to settle into an appropriate alignment with respect to the comfort band 1. In the case of an oval security bracelet 11 used in conjunction with a security band comprising the foam element 5a as illustrated in figure 7a, the major diametric axis of the security bracelet 11 will align itself with the thicker regions 25, and the minor diametric axis of the security bracelet 11 will align itself with the thinner regions 27.

The same principle applies equally in the case of a simple comfort band 1 without electronics to assure the alignment of an oval security bracelet 11 for the purpose of improving the comfort of the wearer.

Although the invention has been described in terms of specific embodiments, variations thereto are possible without departing from the scope of the invention as defined in the appended claims.

## Claims

1. Kit of parts comprising an electronic surveillance bracelet (11) and a comfort band (1) adapted to be worn between the electronic surveillance bracelet (11) and a leg of a wearer, the comfort band (1) comprising:
- a tubular elastic element (3) adapted to retain the comfort band (1) on the leg of the wearer;
- a cushion (5) provided along an edge of the elastic element (3) intended to be disposed towards the foot of the wearer, the cushion (5) extending outwardly from the elastic element (3) and arranged so as to form an abutment for an end face (11a) of the electronic surveillance bracelet (11).

2. Kit of parts according to claim 1, wherein the elastic element (3) has a height (h2) at least corresponding to the height (h1) of the electronic surveillance bracelet (11).

3. Kit of parts according to any preceding claim, wherein the elastic element (3) forms a lip (3a) extending under the cushion (5), the lip (3a) being arranged to urge the cushion (5) in an outward direction.

4. Kit of parts according to any preceding claim, wherein the comfort band (1) comprises a closure (7) to permit the comfort band (1) to be opened and re-closed, the closure (7) preferably comprising a hook and loop fastener.

5. Kit of parts according to any preceding claim, wherein at least one of the elastic element (3) and the cushion (5) comprises at least one physiological property sensor (13) arranged to be in contact with the skin of the wearer.

6. Kit of parts according to claim 5, wherein the physiological property sensor (13) comprises at least one of:
- a heart rate sensor;
- a blood oximetry sensor;
- a skin temperature sensor;
- a perspiration sensor;
- a chemical sensor for detecting a foreign substance such as alcohol, pharmaceuticals or drugs;
- an identification sensor, such as an ultrasonic sensor for measuring a property of the structure of the wearer's ankle.

7. Kit of parts according to one of claims 5 or 6, further comprising at least one electrical contact pad (15) adapted to cooperate with a corresponding electrical contact (17) provided on the electronic surveillance bracelet (11).

8. Kit of parts according to claim 7, wherein the electrical contact pad (15) is provided on a surface of the cushion (5) arranged to face an end surface (11a) of the electronic surveillance bracelet (11).

9. Kit of parts according to one of claims 7 and 8, wherein the electrical contact pad (15) is provided on an outward-facing surface of the elastic element (3).

10. Kit of parts according to one of claims 7-9, wherein the cushion (5) is shaped so as to attain an alignment between the electrical contact pad (15) provided on the comfort band (1) and the corresponding electrical contact (17) provided on the electronic surveillance bracelet (11).

11. Kit of parts according to one of claims 5 or 6, further comprising at least one first antenna (21) adapted to communicate with a corresponding second antenna (23) provided on or in the electronic surveillance bracelet (11).

12. Kit of parts according to claim 11, wherein said first antenna (21) is adapted to transmit data from the physiological property sensor (13) to the electronic surveillance bracelet (11), and/or to power the physiological property sensor (13) wirelessly.

13. Kit of parts according to one of claims 11-12, wherein the cushion (5) is shaped so as to attain an alignment between the first antenna (21) comprised by the comfort band (1) and the second antenna (23) provided on or in the electronic surveillance bracelet (11).

14. Kit of parts according to one of claims 10 and 13, wherein the cushion (5) comprises at least one thicker section (25) and at least one thinner section (27) arranged such that the electronic surveillance bracelet (11) achieves said alignment as a result of movement of the wearer's leg.

## Patentansprüche

1. Teilesatz, der einen elektronischen Überwachungsreif (11) und ein Komfortband (1), das angepasst ist, zwischen dem elektronischen Überwachungsreif (11) und einem Bein eines Trägers getragen zu werden, umfasst, wobei das Komfortband (1) Folgendes umfasst:
- ein rohrförmiges elastisches Element (3), das angepasst ist, das Komfortband (1) am Bein des Trägers zu halten;
- ein Polster (5), das entlang einer Kante des elastischen Elements (3) bereitgestellt ist und zum Fuß des Trägers hin angeordnet sein soll, wobei sich das Polster (5) vom elastischen Element (3) nach außen erstreckt und derart angebracht ist, dass es für eine Stirnseite (11a) des elektronischen Überwachungsreifs (11) einen Anschlag bildet.

2. Teilesatz nach Anspruch 1, wobei das elastische Element (3) eine Höhe (h2) aufweist, die mindestens der Höhe (h1) des elektronischen Überwachungsreifs (11) entspricht.

3. Teilesatz nach einem der vorhergehenden Ansprüche, wobei das elastische Element (3) eine Lippe (3a) bildet, die sich unter das Polster (5) erstreckt, wobei die Lippe (3a) derart angebracht ist, dass sie das Polster (5) in eine Auswärtsrichtung drängt.

4. Teilesatz nach einem der vorhergehenden Ansprüche, wobei das Komfortband (1) einen Verschluss (7) umfasst, um es zu erlauben, dass das Komfortband (1) geöffnet und wieder geschlossen wird, wobei der Verschluss (7) vorzugsweise ein Haken-und-Schlaufe-Befestigungselement umfasst.

5. Teilesatz nach einem der vorhergehenden Ansprüche, wobei mindestens eines des elastischen Elements (3) und des Polsters (5) mindestens einen physiologischen Eigenschaftssensor (13) umfasst, der angebracht ist, um mit der Haut des Trägers in Kontakt zu sein.

6. Teilesatz nach Anspruch 5, wobei der physiologische Eigenschaftssensor (13) mindestens eines von Folgendem umfasst:
- einen Herzfrequenzsensor;
- einen Blutoximetriesensor;
- einen Hauttemperatursensor;
- einen Perspirationssensor;
- einen chemischen Sensor zum Detektieren einer fremden Substanz, wie etwa Alkohol, Medikamente oder Drogen;
- einen Identifikationssensor, wie etwa einen Ultraschallsensor zum Messen einer Eigenschaft der Struktur des Fußgelenks des Trägers.

7. Teilesatz nach einem der Ansprüche 5 oder 6, der ferner mindestens eine Kontaktauflage (15) umfasst, die angepasst ist, mit einem entsprechenden elektrischen Kontakt (17), der am elektronischen Überwachungsreif (11) bereitgestellt ist, zusammenzuwirken.

8. Teilesatz nach Anspruch 7, wobei die elektrische Kontaktauflage (15) auf einer Fläche des Polsters (5) bereitgestellt ist, die derart angebracht ist, dass sie einer Endfläche (11a) des elektronischen Überwachungsreifs (11) zugewandt ist.

9. Teilesatz nach Anspruch 7 und 8, wobei die elektrische Kontaktauflage (15) auf einer nach außen weisenden Fläche des elastischen Elements (3) bereitgestellt ist.

10. Teilesatz nach einem der Ansprüche 7-9, wobei das Polster (5) derart geformt ist, dass zwischen der elektrischen Kontaktauflage (15), die am Komfortband (1) bereitgestellt ist, und dem entsprechenden elektrischen Kontakt (17), der am elektronischen Überwachungsreif (11) bereitgestellt ist, eine Ausrichtung erreicht wird.

11. Teilesatz nach einem der Ansprüche 5 oder 6, der ferner mindestens eine erste Antenne (21) umfasst, die angepasst ist, mit einer entsprechenden zweiten Antenne (23), die am oder im elektronischen Überwachungsreif (11) bereitgestellt ist, zu kommunizieren.

12. Teilesatz nach Anspruch 11, wobei die erste Antenne (21) angepasst ist, Daten vom physiologischen Eigenschaftssensor (13) zum elektronischen Überwachungsreif (11) zu übertragen und/oder den physiologischen Eigenschaftssensor (13) drahtlos mit Strom zu versorgen.

13. Teilesatz nach einem der Ansprüche 11-12, wobei das Polster (5) derart geformt ist, dass zwischen der ersten Antenne (21), die im Komfortband (1) umfasst ist, und der zweiten Antenne (23), die am oder im elektronischen Überwachungsreif (11) bereitgestellt ist, eine Ausrichtung erreicht wird.

14. Teilesatz nach einem der Ansprüche 10 und 13, wobei das Polster (5) mindestens einen dickeren Abschnitt (25) und mindestens einen dünneren Abschnitt (27) umfasst, die derart angebracht sind, dass der elektronische Überwachungsreif (11) die Ausrichtung als ein Resultat der Bewegung des Beins des Trägers erreicht.

## Revendications

1. Jeu de pièces comprenant un bracelet de surveillance électronique (11) et une bande de confort (1) adaptée pour être portée entre le bracelet de surveillance électronique (11) et une jambe d'un porteur, la bande de confort (1) comprenant :
- un élément élastique (3) tubulaire adapté pour retenir la bande de confort (1) sur la jambe du porteur ;
- un coussin (5) prévu le long d'un bord de l'élément élastique (3) destiné à être disposé vers le pied du porteur, le coussin (5) s'étendant vers l'extérieur à partir de l'élément élastique (3) et étant agencé de manière à former une butée pour une face d'extrémité (11a) du bracelet de surveillance électronique (11).

2. Jeu de pièces selon la revendication 1, dans lequel l'élément élastique (3) a une hauteur (h2) correspondant au moins à la hauteur (h1) du bracelet de surveillance électronique (11).

3. Jeu de pièces selon l'une quelconque des revendications précédentes, dans lequel l'élément élastique (3) forme une lèvre (3a) s'étendant sous le coussin (5), la lèvre (3a) étant agencée pour pousser le coussin (5) dans une direction vers l'extérieur.

4. Jeu de pièces selon l'une quelconque des revendications précédentes, dans lequel la bande de confort (1) comprend une fermeture (7) pour permettre à la bande de confort (1) d'être ouverte et refermée, la fermeture (7) comprenant de préférence une attache à boucle et crochet.

5. Jeu de pièces selon l'une quelconque des revendications précédentes, dans lequel au moins l'un parmi l'élément élastique (3) et le coussin (5) comprend au moins un capteur de propriétés physiologiques (13) agencé pour être en contact avec la peau du porteur.

6. Jeu de pièces selon la revendication 5, dans lequel le capteur de propriétés physiologiques (13) comprend au moins l'un parmi :
- un capteur de rythme cardiaque ;
- un capteur d'oxymétrie sanguine ;
- un capteur de température de la peau ;
- un capteur de transpiration ;
- un capteur chimique pour détecter une substance étrangère telle que l'alcool, les produits pharmaceutiques ou les drogues ;
- un capteur d'identification, tel qu'un capteur à ultrasons pour mesurer une propriété de la structure de la cheville du porteur.

7. Jeu de pièces selon l'une des revendications 5 ou 6, comprenant en outre au moins un plot de contact électrique (15) adapté pour coopérer avec un contact électrique correspondant (17) prévu sur le bracelet de surveillance électronique (11).

8. Jeu de pièces selon la revendication 7, dans lequel le plot de contact électrique (15) est prévu sur une surface du coussin (5) agencée pour faire face à une surface d'extrémité (11a) du bracelet de surveillance électronique (11).

9. Jeu de pièces selon l'une des revendications 7 et 8, dans lequel le plot de contact électrique (15) est prévu sur une surface tournée vers l'extérieur de l'élément élastique (3).

10. Jeu de pièces selon l'une des revendications 7 à 9, dans lequel le coussin (5) est formé de manière à obtenir un alignement entre le plot de contact électrique (15) prévu sur la bande de confort (1) et le contact électrique correspondant (17) prévu sur le bracelet de surveillance électronique (11).

11. Jeu de pièces selon l'une des revendications 5 ou 6, comprenant en outre au moins une première antenne (21) adaptée pour communiquer avec une seconde antenne (23) correspondante prévue sur ou dans le bracelet de surveillance électronique (11).

12. Jeu de pièces selon la revendication 11, dans lequel ladite première antenne (21) est adaptée pour transmettre des données, du capteur de propriétés physiologiques (13) au bracelet de surveillance électronique (11), et/ou pour alimenter le capteur de propriétés physiologiques (13) sans fil.

13. Jeu de pièces selon l'une des revendications 11 et 12, dans lequel le coussin (5) est formé de manière à obtenir un alignement entre la première antenne (21) comprise dans la bande de confort (1) et la seconde antenne (23) prévue sur ou dans le bracelet de surveillance électronique (11).

14. Jeu de pièces selon l'une des revendications 10 et 13, dans lequel le coussin (5) comprend au moins une section plus épaisse (25) et au moins une section plus fine (27) agencées de sorte que le bracelet de surveillance électronique (11) réalise ledit alignement suite à un mouvement de la jambe du porteur.
